# EUROPEAN PATENT APPLICATION

(11) **EP 4 844 430 A1**
(43) Date of publication of application: **25.11.2026**
(21) Application number: 25177336.2
(22) Date of filing: 19.05.2025
(51) Int. Cl.: G01R 33/38, G01R 33/48, A61N 5/10, G01R 33/3815

(54) **CRYOSTAT FOR MRI RADIATION THERAPY SYSTEM**

(71) Applicant: Koninklijke Philips N.V., 5656 AE Eindhoven (NL)
(72) Inventor: FORTHMANN, Peter, Eindhoven (NL); AMTHOR, Thomas Erik, Eindhoven (NL)
(74) Representative: Philips Intellectual Property & Standards

(57) **Abstract**

A cryostat (10) for cooling a magnet in a magnetic resonance imager (110) of a magnetic resonance imaging radiation therapy system (100) is disclosed. The cryostat (10) includes an outer vacuum container with a vacuum tight wall (20) that encloses a vacuum region (30). The vacuum tight wall (20) features a beam window portion (22) arranged to intersect with a radiation therapy beam (50). The beam window portion (22) comprises a hollow section (24) of the vacuum tight wall (20) and an internal mechanical support structure (26) located within the hollow section (24), enabling reduced interference with the radiation therapy beam (50) while maintaining structural integrity and vacuum conditions.

## Description

### FIELD OF THE INVENTION

The invention relates to a cryostat for cooling a magnet of a magnetic resonance imager in a magnetic resonance imaging radiation therapy system, and to a magnetic resonance imaging radiation therapy system comprising such a cryostat.

### BACKGROUND OF THE INVENTION

Magnetic resonance imaging radiation treatment systems integrate high-quality, real-time imaging with precision radiotherapy, offering clinicians a dynamic method to monitor and adjust treatment. In such systems, the suitable environment for superconducting magnet operation is combined with the delivery of therapeutic radiation beams, thereby ensuring that imaging and treatment are conducted concurrently on one platform.

US2014/0135615A1 describes a combined MRI and radiation therapy system that integrates MRI imaging equipment with radiation therapy equipment. The MRI system includes a shielded solenoidal magnet with main magnet coils arranged coaxially along an axis, and a shielding arrangement positioned at a greater radius than the main magnet coils. The radiation therapy equipment features a LINAC assembly, which includes a linear electron accelerator with an electron beam path parallel to the axis, an electron beam deflection arrangement, and a target for generating therapeutic radiation.

Cooling of the superconducting magnet is of great importance. However, the cryostat suitable to maintain these conditions may absorb and scatter the treatment beam, potentially reducing both the intensity and uniformity of the radiation that eventually reaches the target area.

Hence, there is a need for an improved configuration that not only maintains the suitable conditions for magnet operation but also minimizes the interaction with the therapeutic beam.

### SUMMARY OF THE INVENTION

It is an object of the invention to provide an improved cryostat structure that limits beam absorption while maintaining mechanical strength and thermal insulation.

The invention is defined by the independent claims. Advantageous embodiments are defined in the dependent claims.

According to a first aspect, there is provided a cryostat for cooling a magnet of a magnetic resonance imaging radiation therapy system. The cryostat comprises an outer vacuum container with a vacuum tight wall enclosing a vacuum region. The vacuum tight wall includes a beam window portion arranged to intersect with a radiation therapy beam. The beam window portion comprises a hollow portion of the vacuum tight wall and an internal mechanical support structure inside the hollow portion. The mechanical support structure, e.g. comprising beams or plates or other supporting elements extending through the thickness of the hollow portion, is configured to provide mechanical support, but takes up only a part of the hollow volume, thereby providing for reduced density as compared to a solid wall. When arranged in the magnetic resonance imaging radiation therapy system, this configuration of the cryostat allows the radiation therapy beam to pass through the cryostat with reduced interference, while maintaining the structural integrity and vacuum environment suitable for the magnet.

In an embodiment, the mechanical support structure is a honeycomb structure. The honeycomb design reduces material density, thereby minimizing beam absorption, while providing sufficient mechanical strength to support the cryostat.

In an embodiment, the mechanical support structure in a 3D-printed structure. 3D printing, or additive manufacturing, allows for precise customization of the support structure's geometry, e.g. enabling optimization of strength and beam transparency.

In an embodiment, the beam window portion is made of composite and/or plastic materials. These materials may have lower radiation absorption compared to metals, reducing beam attenuation while maintaining adequate mechanical and thermal properties.

In an embodiment, at least part of the vacuum tight wall comprises a material different from the beam window portion. The part of the vacuum tight wall that is not the beam window portion may have a material primarily optimized for parameters such as mechanical strength, vacuum, thermal properties etc. and may have a higher beam attenuation than the material in the beam window portion.

In an embodiment, at least part of the vacuum tight wall is a solid wall. In this way, the wall may provide for enhanced mechanical strength and vacuum containment in regions not intersected by the radiation therapy beam.

In an embodiment, the thickness and/or shape and/or material of the mechanical support structure is configured to provide equal absorption of the radiation therapy beam at each of multiple positions along the length and/or width of the beam window portion. This provides for uniform beam intensity across the target area, improving the precision and effectiveness of radiation therapy.

In an embodiment, the thickness and/or shape and/or material of the support structure varies with distance from a center position, where the center position is arranged to correspond to the center of the radiation therapy beam passing through the beam window portion. The thickness and/or shape and/or material of the support structure may be configured such that the radiation therapy beam absorption remains constant for different incident angles of beam paths at corresponding distances from the center position. In this way, uniformity can be improved when the radiation therapy beam comprises non-parallel beams.

In an embodiment, the beam window portion is in the form of a strip extending along a circumference of the vacuum tight wall. A circumferential strip allows the cryostat to accommodate radiation therapy beams from multiple angles along the circumference of the cryostat, enabling greater flexibility in beam delivery.

In an embodiment, the beam window portion comprises a pumping port for pressure regulation. A dedicated pumping port may be used with a vacuum pump to maintain vacuum integrity and facilitate pressure adjustments in the region around the beam window.

According to a second aspect of the invention, there is provided a magnetic resonance imaging radiation therapy system comprising a magnetic resonance imager and a radiation therapy device. The magnetic resonance imager is configured to acquire magnetic resonance data from an examination region, and the radiation therapy device is configured to radiate a radiation therapy beam toward the examination region. The magnetic resonance imager includes a magnet and the cryostat according to the first aspect. At least part of the cryostat is arranged between the radiation therapy device and the examination region such that the beam path of the radiation therapy beam intersects with the beam window portion.

In an embodiment, the radiation therapy device is configured to radiate the therapeutic beam along multiple beam paths from multiple positions around the examination region, with the beam window portion arranged to intersect each of the multiple beam paths.

These and other aspects of the invention will be apparent from and elucidated with reference to the embodiments described hereinafter.

### BRIEF DESCRIPTION OF THE DRAWINGS

Fig. 1 shows a magnetic resonance imaging radiation therapy system.
Fig. 2 illustrates a part of a vacuum tight wall of a cryostat.
Fig. 3 illustrates in more detail an example of beam window of a vacuum tight wall.

### DETAILED DESCRIPTION OF EMBODIMENTS

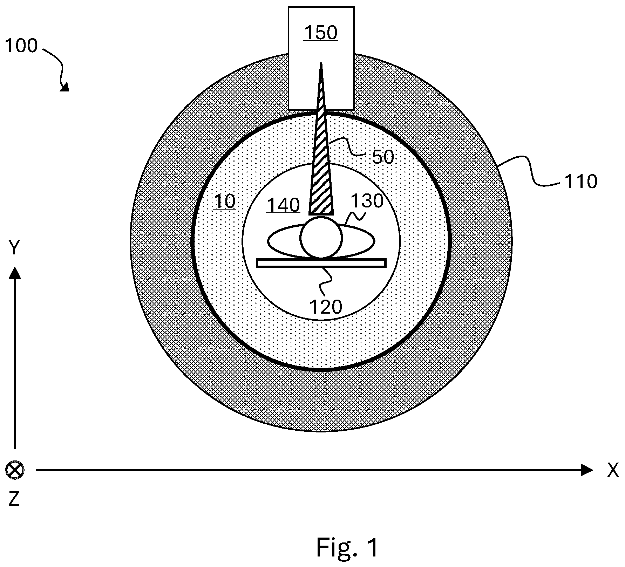

Fig. 1 illustrates a magnetic resonance imaging radiation therapy system 100 that integrates magnetic resonance imaging (MRI) with radiation therapy to enable precise and real-time treatment of a patient 130. The system includes a magnetic resonance imager 110 for imaging the patient 130 in an examination region 140, where the patient 130 is positioned on a patient support 120. The system also includes a radiation therapy device 150, which delivers a radiation therapy beam 50 towards the patient 130.

The magnetic resonance imager 110 includes a cryostat 10 that houses the magnet required for MRI operation. The cryostat 10 features an outer vacuum container with a vacuum tight wall, which encloses a vacuum region to provide thermal insulation for the magnet. The radiation therapy beam 50 from the radiation therapy device 150 passes through the cryostat 10 to reach the patient 130 in the examination region 140. A beam window portion is integrated into the vacuum tight wall of the cryostat 10 at the location where the radiation therapy beam 50 intersects the cryostat. This beam window portion is designed to allow the beam to pass through with reduced absorption and scattering, while maintaining the structural integrity and vacuum environment of the cryostat.

The geometry of the system is shown in the X, Y, and Z directions in the figure. The patient 130 is placed in a bore extending along the Z-direction, which is encircled by the MRI magnet and the cryostat 10. The examination region 140, where imaging and radiation therapy are focused, is located within this bore. The radiation therapy device 150 may be configured to rotate in the Y-X plane, allowing the radiation therapy beam 50 to be delivered from multiple angles around the patient 130. This rotational capability enhances the flexibility of the system, enabling comprehensive coverage of the target area during treatment.

The configuration with a beam window portion ensures that the radiation therapy beam 50 reaches the examination region 140 with little interference, thereby improving the precision and effectiveness of the treatment.

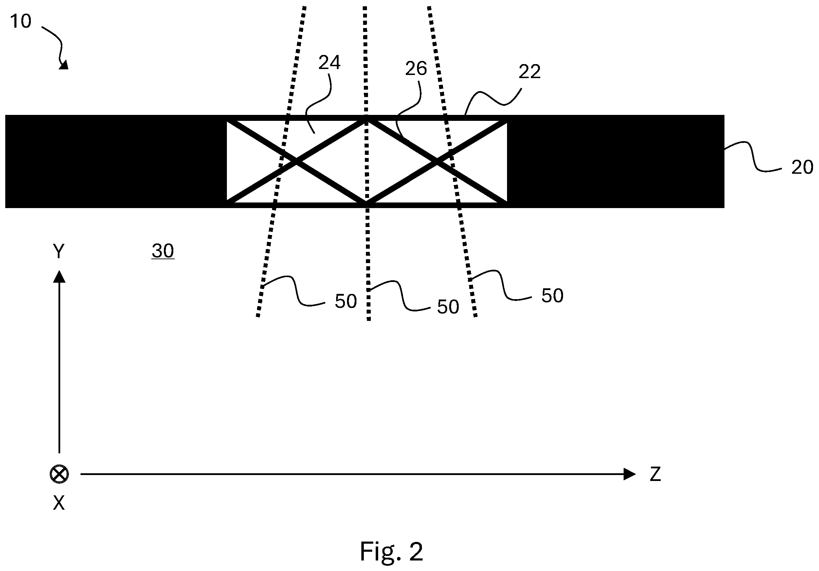

Fig. 2 illustrates a detailed view of the vacuum tight wall 20 of the cryostat 10, with particular reference to the beam window portion 22 through which the radiation therapy beam 50 passes. The beam window portion 22 is designed to minimize interference with the radiation therapy beam 50 while maintaining the vacuum in the vacuum region 30 and the mechanical strength of the cryostat 10. This figure highlights the structural and functional design of the beam window portion 22, demonstrating how it enables the radiation therapy beam 50 to traverse the cryostat 10 with reduced absorption and scattering.

The geometrical axes X, Y, and Z in Fig. 2 align with the same axes shown in Fig. 1. In Fig. 1, the Z-axis runs along the length of the bore of the magnetic resonance imager 110, which is where the patient 130 is positioned in the examination region 140. The X and Y axes define the plane perpendicular to the Z-axis, and the radiation therapy device 150 may rotate in this Y-X plane to deliver the radiation therapy beam 50 from different angles around the patient 130. In Fig. 2, these same axes are used to show the orientation of the vacuum tight wall 20 of the cryostat 10 and the beam window portion 22 where the radiation therapy beam 50 passes through.

The vacuum tight wall 20 encloses a vacuum region 30 that provides thermal insulation for the magnet housed within the cryostat 10. The beam window portion 22 is a specialized segment of the vacuum tight wall 20, configured to intersect with the radiation therapy beam 50. The cross section (X and Z direction) of the beam window is larger than that of the radiation therapy beam 50 at the intersection. The beam window portion 22 includes a hollow portion 24, which reduces material density in the beam path to lessen beam absorption and scattering. Within the hollow portion 24, a mechanical support structure 26 is provided to ensure the structural integrity of the cryostat 10 under vacuum conditions.

In some embodiments, the mechanical support structure 26 may be implemented as a honeycomb structure, which reduces material density while maintaining sufficient mechanical strength. The mechanical support structure 26 may be a 3D-printed structure, allowing for precise customization of its geometry to optimize both strength and beam transparency. The beam window portion 22 may comprise composite and/or plastic materials, which have lower radiation absorption compared to metals, further reducing beam attenuation while maintaining adequate mechanical and thermal properties.

In other embodiments, the thickness and/or shape and/or material of the mechanical support structure 26 may be configured to provide equal absorption of the radiation therapy beam 50 along the length and/or width of the beam window portion 22. This ensures uniform beam intensity across the target area, improving the precision and effectiveness of radiation therapy. Additionally, the thickness and/or shape and/or material of the support structure 26 may vary with distance from a center position, where the center position corresponds to the center of the radiation therapy beam 50 passing through the beam window portion 22. This configuration compensates for variations in beam intensity, further enhancing uniformity and treatment accuracy.

The beam window portion 22 may also be in the form of a strip extending along a circumference of the vacuum tight wall 20, around the Z-axis in this example, allowing the cryostat 10 to accommodate radiation therapy beams from multiple angles.

In some embodiments, the beam window portion 22 may include a pumping port for pressure regulation, which helps maintain vacuum integrity and facilitates pressure adjustments in the region around the beam window.

As shown in Fig. 2, the integration of the hollow portion 24 and the mechanical support structure 26 within the beam window portion 22 achieves a balance between reducing beam interference and maintaining the structural, thermal, and mechanical properties of the cryostat 10. These features collectively ensure that the radiation therapy beam 50 can traverse the cryostat 10 with low attenuation, thereby preserving the intensity and uniformity of the beam as it reaches the target area.

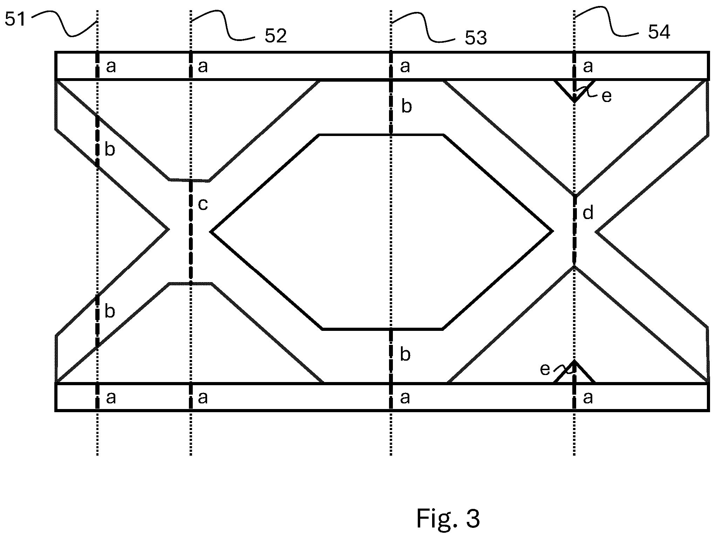

Fig. 3 illustrates an embodiment of the beam window portion 22 designed to achieve equal absorption of a radiation therapy beam along its width. The figure shows four distinct beam paths, namely the first beam path 51, the second beam path 52, the third beam path 53, and the fourth beam path 54, each passing through the beam window portion 22. The beam window portion 22 with the mechanical support structure 26 is structured to ensure that radiation absorption is uniform across all these beam paths, thereby maintaining consistent intensity of the radiation therapy beam 50 and improving the precision of the treatment.

The beam window portion 22 in this example includes structural elements labeled as a, b, c, d, and e, which serve to illustrate paths through which the radiation therapy beam 50 interacts with the material of the beam window portion 22. Such structural elements of the beam window portion 22 and mechanical support structure 26 absorb a portion of the radiation as the beam traverses the window. The relationship between these structural elements is exemplified here, such that total absorption for each path is the same. Hence, absorption of elements 2*b = c = (d + 2*e).

This defined relationship, e.g. achieved with thickness and/or material properties, ensures that the total absorption along each beam path 51, 52, 53 and 54 is equivalent. For instance, the first beam path 51 passes through two segments of a and two segments of b, while the second beam path 52 passes through two segments of a and one segment of c. Similarly, the third beam path 53 traverses two segments of a and two segments of b, and the fourth beam path 54 traverses two segments of a, one segment of d, and two segments of e.

It is noted that the therapeutic radiation beams may be divergent, rather than parallel as indicated in the simple example in Fig. 3. Hence, the angle with which each beam traverses the structure may be different and depend on the coordinate in e.g. the Z-direction or X-direction, e.g. vary like in a cone with the distance from a center point of the window. The beam window 22 and mechanical support structure 26 may therefore, in a similar way, be designed taking the angle of the partial beams at different positions into account.

The absorption properties through the beam window portion 22 are in the example in Fig. 3 controlled to be equal at the different locations via the geometry of the mechanical support structure 26, i.e. through thickness variations. The same effect may, alternatively or additionally, be obtained by varying the material properties, such as a composite formulation. If the structure is produced in a 3D printing process, for example, the composition of the material may be controlled with the manufacturing process to vary depending on the position.

A configuration with equal absorption along the width and/or each angle through the beam window is particularly advantageous in maintaining uniform beam intensity across the target area, which is advantageous in achieving effective and precise radiation therapy. By carefully designing the thickness, material and/or arrangement of the structural elements of the mechanical support structure, the beam window portion 22 minimizes variations in beam absorption, even when the beam paths intersect the window at different positions. The geometry and material distribution within the beam window portion 22 may be optimized to provide the desired uniform performance.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

### REFERENCE SIGNS

- 10: Cryostat
- 20: Vacuum tight wall
- 22: Beam window portion
- 24: Hollow portion
- 26: Mechanical support structure
- 30: Vacuum region
- 50: Radiation therapy beam
- 51-54: Beam paths
- 100: Magnetic resonance imaging radiation therapy system
- 110: Magnetic resonance imager
- 120: Patient support
- 130: Patient
- 140: Examination region
- 150: Radiation therapy device
- a-e: Beam absorbing paths

## Claims

1. A cryostat (10) for cooling a magnet of a magnetic resonance imaging radiation therapy system (100),
wherein the cryostat (10) comprises an outer vacuum container with a vacuum tight wall (20) enclosing a vacuum region (30),
wherein the vacuum tight wall (20) has a beam window portion (22) arranged to intersect with a radiation therapy beam (50) in the magnetic resonance imaging radiation therapy system,
wherein the beam window portion (22) comprises a hollow portion (24) of the vacuum tight wall, and
wherein the beam window portion comprises an internal mechanical support structure (26) inside the hollow portion.

2. The cryostat according to claim 1, wherein the mechanical support structure (26) is a honeycomb structure.

3. The cryostat according to claim 1 or 2, wherein the mechanical support structure (26) is a 3D printed structure.

4. The cryostat according to claim 1 or 2 or 3, wherein the beam window portion (22) comprises a composite and/or plastic material.

5. The cryostat according to any of the preceding claims, wherein at least part of the vacuum tight wall (20) comprises a different material than the beam window portion (22).

6. The cryostat according to any of the preceding claims, wherein at least part of the vacuum tight wall (20) is a solid wall.

7. The cryostat according to any of the preceding claims, wherein a thickness and/or shape and/or material of the mechanical support structure (26) at multiple positions along a length and/or width of the beam window portion is configured to provide for equal absorption of the radiation therapy beam (50) passing through the beam window portion (22), at each of the multiple positions.

8. The cryostat according to claim 7, wherein the mechanical support structure (26) has a thickness and/or shape and/or material that varies with distance from a center position, wherein the center position is arranged to correspond to a center of the radiation therapy beam (50) passing through the beam window portion.

9. The cryostat according to any of the preceding claims, wherein the beam window portion (22) is in the form of a strip extending along a circumference of the vacuum tight wall (20).

10. The cryostat according to any of the preceding claims, wherein the beam window portion (22) comprises a pumping port for pressure regulation.

11. A magnetic resonance imaging radiation therapy system (100) comprising:
- a magnetic resonance imager (110) configured to acquire magnetic resonance data from an examination region (140); and
- a radiation therapy device (150) configured to radiate a radiation therapy beam (50) with a beam path towards the examination region (140);
wherein the magnetic resonance imager (110) comprises a magnet,
wherein the magnetic resonance imager comprises the cryostat (10) according to any of the preceding claims, and
wherein at least part of the cryostat (10) is arranged between the radiation therapy device (150) and the examination region (140), such that the beam path of the radiation therapy beam (50) intersects with the beam window portion (22).

12. The system according to claim 11, wherein the radiation therapy device (150) is configured to radiate the therapeutic beam (50) with multiple beam paths from multiple positions around the examination region (140), and wherein the beam window portion (22) is arranged to intersect with each of the multiple beam paths.
